(19) Europäisches Patentamt
European Patent Office
Office européen des brevets

(11) **EP 3 752 590 B1**

(12) **EUROPÄISCHE PATENTSCHRIFT**

(45) Veröffentlichungstag und Bekanntmachung des Hinweises auf die Patenterteilung:
**13.10.2021 Patentblatt 2021/41**

(21) Anmeldenummer: **19724393.4**

(22) Anmeldetag: **09.05.2019**

(51) Int Cl.:
*C12M 1/00* (2006.01)      *C12M 1/12* (2006.01)
*C12M 3/06* (2006.01)

(86) Internationale Anmeldenummer:
**PCT/EP2019/061936**

(87) Internationale Veröffentlichungsnummer:
**WO 2019/238320 (19.12.2019 Gazette 2019/51)**

(54) **VERFAHREN ZUR BEGASUNG VON BIOREAKTOREN SOWIE BEGASUNGSSYSTEM**

METHOD FOR GASSING BIOREACTORS AND GASING SYSTEM

PROCÉDÉ DE GAZAGE DE BIORÉACTEURS ET SYSTÈME GAZEUX

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.06.2018 DE 102018114414**

(43) Veröffentlichungstag der Anmeldung:
**23.12.2020 Patentblatt 2020/52**

(73) Patentinhaber: **Adolf Kühner AG**
**4127 Birsfelden (CH)**

(72) Erfinder:
• **ANDERLEI, Tibor**
**79379 Müllheim (DE)**
• **ZUMBRUNNEN, Simon Paul**
**3414 Oberburg (CH)**
• **SCHÄR, Manfred**
**3400 Burgdorf (CH)**
• **RICHTER, Andreas**
**4133 Pratteln (CH)**

(74) Vertreter: **Kohlmann, Kai**
**Donatusstraße 1**
**52078 Aachen (DE)**

(56) Entgegenhaltungen:
EP-A1- 0 965 632      EP-A1- 2 975 110
WO-A1-2007/116266

**Beschreibung**

Die Erfindung betrifft ein Verfahren zur Begasung von mehreren Bioreaktoren sowie ein Begasungssystem zur Durchführung des Verfahrens.

Eine kontrollierte Begasung mit verschiedenen Gasen oder Gasgemischen ist insbesondere bei der automatisierten Erfassung von Prozessparametern mikrobieller, biochemischer, enzymatischer und chemischer Reaktionen in Reaktionsflüssigkeiten in Bioreaktoren erforderlich, die bis zum Abschluss der Reaktion in sämtlichen Mikroreaktoren unterbrechungslos geschüttelt werden können. Als Parameter der Reaktionsflüssigkeiten werden beispielsweise die Sauerstoff-Transferrate (OTR) und die Kohlendioxid-Transferrate (CTR) erfasst und daraus die Parameter Respirationsquotient (RQ) und maximale spezifische Wachstumsrate ($\mu_{max}$) abgeleitet.

Aus dem Stand der Technik ist ein Universal-Messsystem zur Bioprozessoptimierung in Schüttelkolben durch Ermittlung der vorgenannten Prozessparameter bekannt (vgl. Hitec-Zang, Bioprozessoptimierung in Schüttelkolben, heruntergeladen am 16.05.2018 unter https://www.hitec-zang.de/fileadmin/ informationsmaterial/flyer_deu/web/RAMOS_Flyer_2013_web.pdf).

Mit dem Universal-Messsystem können Parallelversuche in acht Bioreaktoren durchgeführt und Atmungsaktivitäten erfasst werden. Zur übereinstimmenden Begasung der Kulturflüssigkeiten in den Bioreaktoren wird ein konstanter Gasstrom auf mehrere Gasleitungen verteilt, die mit einem Gaseintritt an jedem Bioreaktor verbunden sind. Um die acht Messkolben mit übereinstimmenden Teilgasströmen zu begasen, kommen Lochblenden als örtlicher Strömungswiderstand zum Einsatz, die den Leitungsquerschnitt sprungartig verengen. Für eine gleichmäßige Verteilung des Gases ist ein relativ hoher Druckverlust an den Lochblenden >200 mbar erforderlich. Dieser relativ hohe Druckverlust an den Lochblenden gewährleistet, dass kleine Druckverluste aufgrund unterschiedlicher Länge der Gasleitungen zu den Bioreaktoren und/ oder an Abgasfiltern nahezu keinen Einfluss auf die gleichmäßige Verteilung des Gases auf sämtliche Bioreaktoren haben. Das bekannte Verfahren zur gleichmäßigen Begasung von mehreren Bioreaktoren bedingt höhere Drücke in der Gasversorgung sowie höhere Sicherheitsmaßnahmen beim Einsatz von Befeuchtungsflaschen für das Gas, die auf die höheren Drücke ausgelegt werden müssen.

Des Weiteren bietet die Firma Eppendorf aus Hamburg unter der Bezeichnung DASGIP MX4/4 ein Gasmischmodul an, das vier separate Bioreaktoren mit unabhängig zusammengestellten Mischungen aus Luft, Stickstoff, Sauerstoff und Kohlendioxid versorgt. Jeder Gasausgang hat eigene Soll-Werte für die Flussrate sowie die $O_2$- und $CO_2$-Konzentration. Das Gasmischmodul der Firma Eppendorf erfordert den Einsatz von Ventilen und einer komplexen Steuerungselektronik. Eine Befeuchtung des Gasstroms erfordert eine gesonderte Befeuchtungsstrecke für jeden der vier Bioreaktoren (vgl. Eppendorf, DASGIP® MX-Module für massendurchflussgeregeltes Gasmischen, heruntergeladen am 16.05.2018 unter https://online-shop.eppendorf.de/DE-de/Bioprozesstechnik-44559/Module-77460/DASGIP-MX-Module-fuermassendurchflussgeregeltes-Gasmischen-PF-60977.html?_ga=2.188935403.1365824566.15264885 98-1728287905.1526488598).

Die EP2975110 A1 offenbart ein Begasungssystem sowie ein Verfahren zur Begasung mehrerer, parallel angeordneter Bioreaktoren (201), wobei das Verfahren die Bereitstellung eines konstanten Gasstroms, der Sauerstoff und Kohlendioxid enthält, umfaßt, gefolgt von einem Schritt des Zuführens des konstanten Gasstroms in einen Behälter mit einer Flüssigkeitsfüllung, das Aufteilen des Gasstroms in mehrere Teilgasströme sowie das Einleiten jedes Teilgasstroms über eine untere Öffnung in die Vielzahl an Bioreaktoren.

Die WO 2007/116266 A1 offenbart ein Verfahren zur Begasung mehrerer Bioreaktoren mit einem konstanten Gasstrom aus einer Druckluftquelle. Der Gasstrom durchläuft zunächst eine Befeuchtungsflasche und wird anschließend mittels mehrerer T-Stücke in einer Zufuhrleitung in Teilgasströme aufgeteilt, die über von den T-Stücken abzweigende Gasleitungen in jeweils einen der Bioreaktoren eingeleitet werden. Um im Wesentlichen gleiche Begasungsbedingungen in jedem Bioreaktor zu schaffen, werden Gegendruck erzeugende Mittel, wie flussregulierende Ventile oder Düsen vorgeschlagen, die vor oder hinter jedem Bioreaktor angeordnet sind, die jeweils einen Gegendruck erzeugen, der größer ist als der Widerstand des Gasstroms zwischen den Gegendruck erzeugenden Mitteln. Ausgehend von diesem Stand der Technik liegt der Erfindung die Aufgabe zugrunde, ein Verfahren zur Begasung von mehreren Bioreaktoren sowie ein Begasungssystem zu schaffen, das ohne eine gesonderte Befeuchtungsvorrichtung einen konstanten Gasstrom mit hoher Verteilgenauigkeit in mehrere Teilgasströme mit einem vorgegebenen Volumenstrom aufteilt, der selbst bei einem während der Begasung schwankenden Gegendruck in der Gasleitung zu dem jeweiligen Bioreaktor auf dem vorgegebenen Niveau konstant gehalten werden kann. Insbesondere liegt der Erfindung die Aufgabe zugrunde, einen konstanten Gasstrom in mehrere Teilgasströme mit übereinstimmendem Volumenstrom weitgehend unabhängig vom Gegendruck in den Gasleitungen zu den einzelnen Bioreaktoren aufzuteilen.

Die Lösung dieser Aufgabe beruht auf dem Gedanken, die Gasverteilung von dem Gegendruck durch einen hydrostatischen Druckausgleich zu entkoppeln, wobei die Gasverteilung zugleich eine zwangsläufige Befeuchtung des Gasstroms bewirkt. Im Einzelnen wird die Aufgabe durch ein Verfahren mit den Merkmalen des Anspruchs 1 sowie ein Begasungssystem mit den Merkmalen des Anspruchs 6 gelöst. Vorteilhafte Ausgestaltungen der Erfindung ergeben sich aus den Unteransprüchen.

Durch das Aufteilen des Gasstroms in mehrere Teilgasströme und das Einleiten jedes Teilgasstroms in die Flüssigkeitsfüllung, wird das Gas zwangsläufig befeuchtet.

Das Einleiten jedes Teilgasstroms in die Flüssigkeitsfüllung kann jeweils unterhalb einer der Steigleitungen erfolgen. Das Einleiten jedes Teilgasstroms in eine der separaten, eine untere Öffnung aufweisenden Steigleitungen erfolgt in diesem Fall vorzugsweise mittels eines unterhalb der Steigleitungen angeordneten Verteilers, aus dem die einzelnen Teilgasströme in Form von Gasblasen austreten und in den Steigleitungen aufsteigen. Dem Verteiler wird der Gasstrom über eine Zufuhrleitung zugeführt.

Alternativ kann das Einleiten jedes Teilgasstroms in die Flüssigkeitsfüllung innerhalb einer der Steigleitungen erfolgen. Das Einleiten jedes Teilgasstroms in eine der separaten, eine untere Öffnung aufweisenden Steigleitungen erfolgt in diesem Fall vorzugsweise mittels eines Verteilers, dessen Austrittsöffnungen für die Teilgasströme innerhalb der Steigleitungen münden. Aus den Austrittsöffnungen treten die einzelnen Teilgasströme in Form von Gasblasen aus und steigen innerhalb der Flüssigkeitsfüllung in den Steigleitungen auf. Dem Verteiler wird der Gasstrom über eine Zufuhrleitung zugeführt.

Nach dem Pascal'schen Gesetz wird die Gasverteilung von dem Gegendruck durch hydrostatischen Druckausgleich wie folgt entkoppelt:

$$p(h) = \rho g h + p_0$$

mit:

p(h) = Hydrostatischer Druck in Abhängigkeit von der Höhe h des Flüssigkeitsspielgels innerhalb der Steigleitung
p = Dichte der Flüssigkeit
g = Erdbeschleunigung
h = Flüssigkeitsspiegel innerhalb der Steigleitung über der unteren Öffnung der Steigleitung
$p_0$ = Druck auf die Flüssigkeitsoberfläche in der Steigleitung

Erhöht sich beispielsweise der Druck in einer Gasleitung durch einen verstopften Abgasfilter an dem Bioreaktor, erhöht sich der Druck $p_0$ auf die Flüssigkeitsoberfläche in der betreffenden Steigleitung. Der steigende Druck auf die Flüssigkeitsoberfläche bewirkt, dass der Flüssigkeitsspiegel in der an die Gasleitung angeschlossenen Steigleitung gegenüber dem Flüssigkeitsspiegel in dem Behälter sinkt. Der steigende Druck $p_0$ auf die Flüssigkeitsoberfläche wird nach dem Pascal'schen Gesetz durch die geringere Höhe h des Flüssigkeitsspiegels über der unteren Öffnung der Steigleitung ausgeglichen. Der Druck des Teilgasstroms an dem Verteiler bleibt indes konstant und tritt gegen einen unveränderten hydrostatischen Druck mit unverändertem Volumenstrom in die Steigleitung ein.

Ein weiterer Vorteil der Erfindung besteht darin, dass sich ein erhöhter Druck $p_0$ auf die Flüssigkeitsoberfläche in einer der Steigleitungen, beispielsweise wegen eines verstopften Abgasfilters, nicht auf die Begasung der übrigen Bioreaktoren auswirkt.

In einer vorteilhaften Ausgestaltung der Erfindung wird der konstante Gasstrom mittels des Verteilers in Teilgasströme mit übereinstimmendem Volumenstrom aufgeteilt. Unabhängig von dem Gegendruck in den an die Steigleitungen angeschlossenen Gasleitungen werden aufgrund des hydrostatischen Druckausgleichs sämtliche Bioreaktoren mit übereinstimmendem Volumenstrom begast.

Sofern das Einleitung der Teilgasströme in die Flüssigkeitsfüllung unterhalb der Steigleitungen erfolgt, ist der Verteiler vorzugsweise in einem geringen Abstand unterhalb der Steigleitungen derart angeordnet, dass die aus dem Verteiler austretenden Teilgasströme möglichst vollständig in die Steigleitungen eingeleitet werden, insbesondere auch dann wenn die Bioreaktoren geschüttelt werden.

Die Teilgasströme werden vorzugsweise in übereinstimmendem vertikalem Abstand zum Flüssigkeitsspiegel in die Steigleitungen eingeleitet. Wenn die Teilgasströme außerdem in übereinstimmendem vertikalem Abstand zum Flüssigkeitsspiegel in die Flüssigkeitsfüllung eingeleitet werden, wird eine Einleitung der Teilgasströme unter übereinstimmenden Bedingungen in sämtlichen Steigleitungen erreicht. Sämtliche Teilgasströme legen eine übereinstimmende Wegstrecke bis zum Eintritt in die jeweilige Steigleitung und/oder innerhalb der Steigleitungen zurück. Sofern die aus dem Verteiler austretenden Teilgasströme einen übereinstimmenden Volumenstrom aufweisen, tritt auch ein übereinstimmender Volumenstrom in jede Steigleitung ein.

Das erfindungsgemäße Verfahren ist insbesondere für eine Begasung von mehreren geschüttelten Bioreaktoren geeignet, da nicht nur die Bioreaktoren, sondern auch der Behälter zur Befeuchtung und Verteilung des Gasstroms aufgrund der ausschließlich mechanischen Komponenten problemlos geschüttelt werden können.

Das vorliegende Begasungssystem weist als wesentliche Komponenten den Behälter, die Steigleitungen und den mit der Zufuhrleitung verbundenen Verteiler auf. Der Behälter ist an seiner Oberseite offen und weist beispielsweise die Form einer Flasche auf. Der Behälter enthält die Flüssigkeitsfüllung, in die die Zufuhrleitung sowie die Steigleitungen eintauchen, wobei an die Zufuhrleitung endseitig der Verteiler fluidleitend angebunden ist. Über die Gasversorgung wird ein konstanter Gasstrom in der Zufuhrleitung bereitgestellt, wobei der Gasstrom lediglich ein Gas, wie beispielsweise Sauerstoff, Kohlendioxid, Stickstoff oder ein Gasgemisch, beispielsweise aus den vorgenannten Gasen oder steril gefilterte Umgebungsluft enthalten kann.

Sofern der Gasstrom eine Gasmischung enthält, weist die Gasversorgung ein Gasmischsystem auf, dessen Eingänge mit mehreren Gasquellen für die unterschiedlichen Gase und dessen Ausgang mit der Zufuhrleitung zum Verteiler verbunden ist. Um das Mischungsverhältnis der einzelnen Gase konstant zu halten, weist das Gasmischsystem vorzugsweise einen Massendurchflussregler (MFC) für jede Gasquelle auf. Mit dem Massendurchflussregler wird der Volumenstrom jedes Gases auf einen Soll-Wert geregelt. Der Massendurchflussregler umfasst üblicherweise einen Massendurchflussmesser, einen über eine Schnittstelle programmierbaren Regler und ein Proportionalventil. Über die Programmierschnittstelle werden für jedes Gas die erforderlichen Kalibrierdaten geladen.

Die Zufuhrleitungen und die Steigleitungen des erfindungsgemäßen Begasungssystems sind aus Stabilitätsgründen zumindest abschnittsweise innerhalb des Behälters als Rohrleitungen ausgeführt, die sich in die Flüssigkeitsfüllung senkrecht zu dessen Flüssigkeitsspiegel erstrecken. Die senkrechte Anordnung ist raumsparend und konstruktiv vorteilhaft, insbesondere wenn die Steigleitungen kreisförmig um die Zufuhrleitung angeordnet sind. Die kreisförmige Anordnung der Steigrohre eliminiert den Einfluss der Schüttelbewegung eines Rotationsschüttlers auf die Gasverteilung in der Flüssigkeit des Behälters, sofern dieser während der Begasung mitgeschüttelt wird.

Ausgestaltungen des Verteilers ergeben sich aus den Merkmalen der Ansprüche 12 und 13:
Die Variante nach Anspruch 12 verteilt den Gasstrom auf die Teilgasströme mit mehreren sich sternförmig nach außen erstreckenden Verteilerrohren. Zur Gasmengenbegrenzung können die Verteilerrohre einen reduzierten Durchmesser (Kappliarrohre) oder endseitig eine Verjüngung aufweisen. Die Gasmengenbegrenzung vergleichmäßigt die Teilgasströme. Der Winkel zwischen den Verteilerrohren entspricht dem Winkel zwischen den kreisförmig um die Zufuhrleitung angeordneten Steigleitungen.

Der Verteiler in der Ausführungsform nach Anspruch 13 weist einen fluidleitend mit der Zufuhrleitung verbundenen hohlzylindrischen Gassammler auf, an dessen oberer Stirnseite mehrere Lochblenden angeordnet sind. Jede Lochblende fluchtet mit einer unteren Öffnung einer der Steigleitungen. Die Lochblenden in dem Gasverteiler bilden einen Strömungswiderstand und dienen damit als Gasmengenbegrenzer zur Vergleichmäßigung der aus dem Verteiler austretenden Teilgasströme.

Wenn die Längsmittelachse der Zufuhrleitung und des hohlzylindrischen Gassammlers zusammenfallen, sämtliche Lochblenden geometrisch übereinstimmen und im gleichen radialen Abstand von der Längsmittelachse angeordnet sind, stimmen die Strömungsverhältnisse an sämtlichen Lochblenden technisch überein. Die übereinstimmenden Strömungsverhältnisse tragen dazu bei, dass an sämtlichen Lochblenden übereinstimmende Teilgasströme austreten.

Um eine Justierung und Ausrichtung des an der Zufuhrleitung angeordneten Verteilers in Bezug auf die Steigleitungen zu vermeiden, weist der Behälter in einer vorteilhaften Ausgestaltung der Erfindung eine mit einem Deckel verschließbare Öffnung auf, wobei die Zufuhrleitung mit dem Verteiler und die Steigleitungen an dem Deckel befestigt sind. Die vorgenannten Komponenten sind als Baugruppe einfach handhabbar. Die Zufuhrleitung mit dem Verteiler und die Steigleitungen lassen sich innerhalb der Flüssigkeitsfüllung durch Verschließen der Behälteröffnung mit dem Deckel lagerichtig positionieren. Für den hydrostatischen Druckausgleich ist es nicht erforderlich, dass der Deckel den Behälter gasdicht verschließt. Um einen starken Druckanstieg in dem Behälter zu vermeiden, kann bei einem den Behälter gasdicht verschließenden Deckel ein Überdruckventil oder Gasfilter an dem Deckel oder dem Behälter vorgesehen sein.

Der an dem Deckel befestigte Abschnitt der Zufuhrleitung und die Steigleitungen sind vorzugsweise als Rohrleitungen ausgeführt. An die obere Öffnung jeder Steigleitung ist gasdicht die Gasleitung angeschlossen, die fluidleitend mit einem Innenraum eines der Bioreaktoren verbunden ist. Um einen hohen Druck innerhalb der Bioreaktoren zu vermeiden, weist jeder Bioreaktor vorzugsweise ein Überdruckventil und / oder einen Abgasfilter auf, über den ein Druckausgleich stattfinden kann.

Der Behälter und / oder die Steigleitungen bestehen vorzugsweise zumindest teilweise aus transparentem Material, insbesondere aus Glas oder Kunststoff. Das transparente Material erlaubt eine Sichtkontrolle des herrschenden Gegendrucks in den Steigleitungen, indem der Flüssigkeitsspiegel der Flüssigkeitssäule in jeder Steigleitung sichtbar ist.

Nachfolgend wird die Erfindung anhand der Zeichnungen näher erläutert. Es zeigen

**Figur 1** eine schematische Darstellung eines erfindungsgemäßen Begasungssystems zur Begasung von mehreren Bioreaktoren,

**Figur 2** ein Begasungssystem nach Figur 1 mit geschüttelten Bioreaktoren sowie

**Figur 3** eine Ausführungsform eines Behälters zum Verteilen und Befeuchten eines Gasstroms.

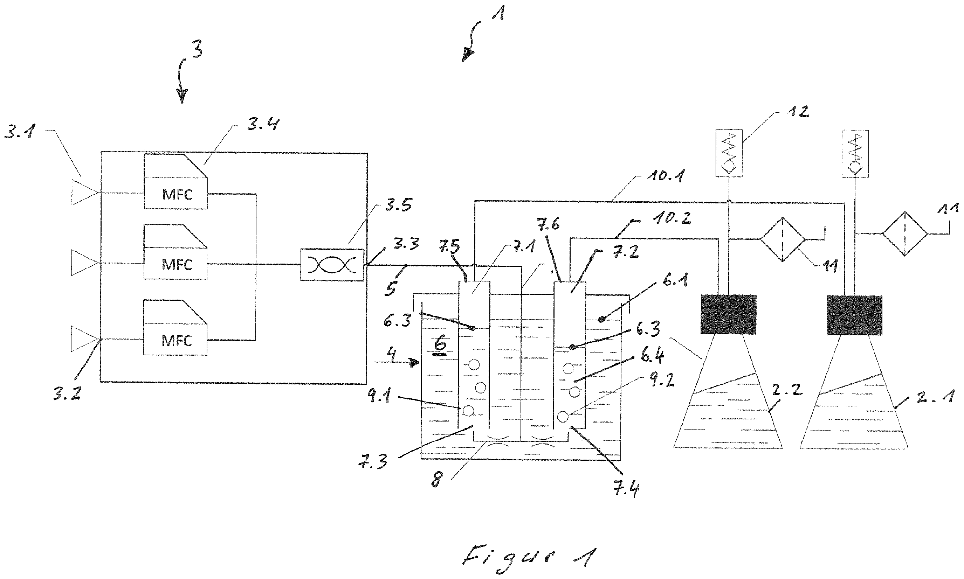

Figur 1 zeigt ein Schema eines Begasungssystems (1) zur Begasung von mehreren Bioreaktoren (2.1, 2.2). In dem Ausführungsbeispiel sind aus Gründen der Übersichtlichkeit lediglich zwei Bioreaktoren dargestellt. Mit dem erfindungsgemäßen Begasungssystem lassen sich jedoch ohne weiteres mehr als zwei, beispielsweise sechs Bioreaktoren zugleich begasen. Die Bioreaktoren (2.1, 2.2) sind zur Aufnahme einer Biomasse, eines flüssigen Nährmediums sowie zur Einleitung der Begasung eingerichet.

Als weitere wesentliche Komponenten umfasst das Begasungssystem (1) eine Gasversorgung (3) zur Bereitstellung eines konstanten Gasstroms, der in dem Ausführungsbeispiel ein Gemisch aus drei Gasen enthält und einen Behälter (4), eingerichtet zum Befeuchten und Aufteilen des Gasstroms in mehrere Teilgasströme.

Der Behälter (4) enthält eine Flüssigkeitsfüllung (6), in die senkrecht zum Flüssigkeitsspiegel (6.1) Steigleitungen (7.1, 7.2) eintauchen. Die Steigleitungen (7.1, 7.2) sind aus Stabilitätsgründen als Rohre ausgeführt und weisen eine übereinstimmende Länge sowie einen übereinstimmenden Durchmesser auf. Eine untere Öffnung (7.3, 7.4) jeder Steigleitung (7.1, 7.2) ist oberhalb eines Verteilers (8) angeordnet, der fluidleitend über eine Zufuhrleitung (5) an die Gasversorgung (3) angeschlossen ist. Der Verteiler (8) teilt den Gasstrom in eine der Anzahl der Bioreaktoren (2.1, 2.2) entsprechende Anzahl an Teilgasströmen (9.1, 9.2) auf, die in Figur 1 durch Luftblasen symbolisiert sind.

Der Verteiler (8) ist in geringem Abstand unterhalb der unteren Öffnungen (7.3, 7.4) der Steigleitungen (7.1, 7.2) angeordnet. Die Austrittsöffnungen des Verteilers (8) können jedoch auch in den Steigleitungen münden, soweit sichergestellt ist, dass die Teilgasströme stets in die Flüssigkeitsfüllung (6) eingeleitet werden. An einer oberen Öffnung (7.5, 7.6) jeder Steigleitung (7.1, 7.2) ist gasdicht eine Gasleitung (10.1. 10.2) angeschlossen, die jeweils eine der Steigleitungen (7.1, 7.2) mit dem Innenraum eines der Bioreaktoren (2.1, 2.2) verbindet.

Aus der schematischen Darstellung ist erkennbar, dass die Gasleitung (10.1) zu dem Bioreaktor (2.1) länger ist, als die Gasleitung (10.2) zu dem Bioreaktor (2.2). Folglich setzt die Gasleitung (10.1) dem Teilgasstrom einen größeren Strömungswiderstand entgegen als die Gasleitung (10.2). Der höhere Strömungswiderstand führt zu einem höheren Druck in der Gasleitung (10.2) als in der Gasleitung (10.1). Der unterschiedliche Gasdruck wirkt auf die Flüssigkeitsoberfläche (6.3) der Flüssigkeitssäule (6.4) in den Steigleitungen (7.1, 7.2). Aufgrund des höheren Drucks in der Gasleitung (10.2) sinkt der Flüssigkeitsspiegel der Flüssigkeitssäule (6.4) in der Steigleitung (7.2) stärker ab, als der Flüssigkeitsspiegel der Flüssigkeitssäule (6.4) in der Steigleitung (7.1).

Der in der Steigleitung (7.2) auf die Flüssigkeitsoberfläche (6.3) wirkende höhere Druck wird durch die geringere Höhe (6.5) der Flüssigkeitssäule (6.4) in der Steigleitung (7.2) kompensiert, sodass der in beiden Steigleitungen (7.1, 7.2) wirksame hydrostatische Druck trotz unterschiedlicher Drücke in den Gasleitungen (10.1, 10.2) im Wesentlichen übereinstimmt.

Die Teilgasströme (9.1, 9.2) treten unbeeinflusst von den Strömungswiderständen in den Gasleitungen (10.1, 10.2) mit übereinstimmendem Volumenstrom aus dem Verteiler (8) aus und in die Flüssigkeitssäulen der Steigleitungen (7.1, 7.2) ein. Aufgrund des übereinstimmenden hydrostatischen Drucks in den Steigleitungen (7.1, 7.2) ist eine gleichmäßige Begasung der beiden Bioreaktoren gewährleistet. Selbst dann, wenn sich beispielsweise ein an einem der Bioreaktoren (2.1, 2.2) zum Druckausgleich angeordneter Gasfilter (11) zusetzen würde, hätte die infolgedessen eintretende Druckerhöhung in der Gasleitung (10.1, 10.2) keinen Einfluss auf den Volumenstrom des dem Bioreaktor (2.1, 2.2) zugeführten Gases.

Im dargestellten Ausführungsbeispiel sollen die Zellkulturen mit übereinstimmenden Teilgasströmen (9.1, 9.2) eines Gasgemisches begast werden. Zu diesem Zweck weist die Gasversorgung (3) ein Gasmischsystem auf, dessen Eingänge (3.2) mit mehreren Gasquellen (3.1) verbunden sind und dessen Ausgang (3.3) mit der Zufuhrleitung (5) verbunden ist. Bei den Gasquellen (3.1) handelt es sich beispielsweise um Druckgasflaschen für Sauerstoff, Kohlendioxid und Stickstoff. Für jede Gasquelle (3.1) weist das Gasmischsystem einen Massendurchflussregler (3.4) auf, der den Volumenstrom des Gases auf einen Soll-Wert regelt. Die zu mischenden Gase werden nach Regelung auf den Soll-Wert einer Mischeinrichtung (3.5) zugeführt, in der die Volumenströme aus Sauerstoff, Kohlendioxid und Stickstoff vermischt werden.

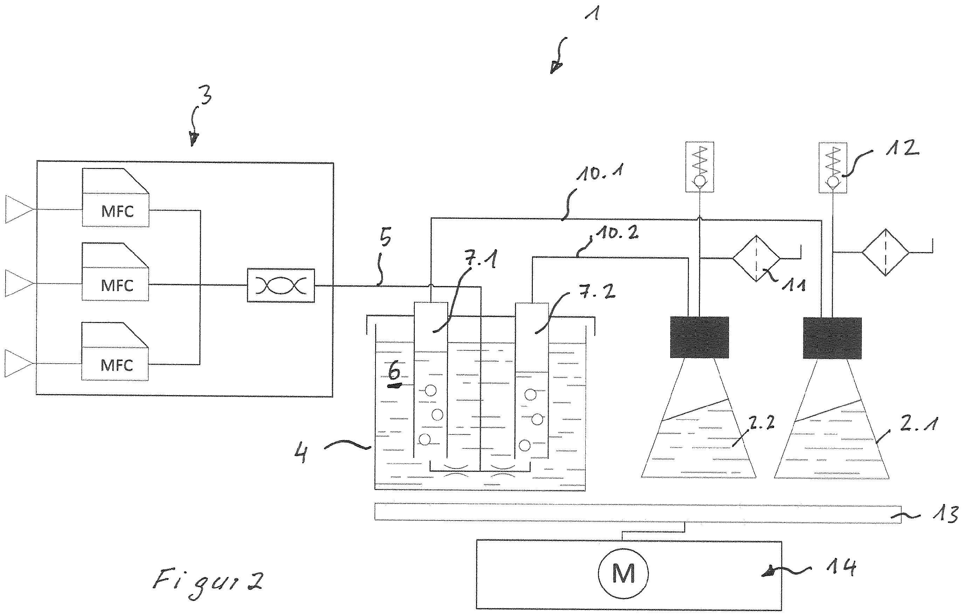

Figur 2 zeigt ein Begasungssystem (1) entsprechend Figur 1, das sich lediglich insoweit unterscheidet, als der Behälter (4) und die Bioreaktoren (2.1, 2.2) nicht stationär, sondern auf einem Tisch (13) eines Rotationsschüttlers (14) angeordnet sind. Die Gasversorgung (3) ist indes unverändert stationär angeordnet. Die Zufuhrleitung (5) muss deshalb bis zu dem als Rohr ausgeführten, in die Flüssigkeitsfüllung (6) eintauchenden Abschnitt flexibel ausgeführt sein, um die Schüttelbewegung während der Begasung zu ermöglichen.

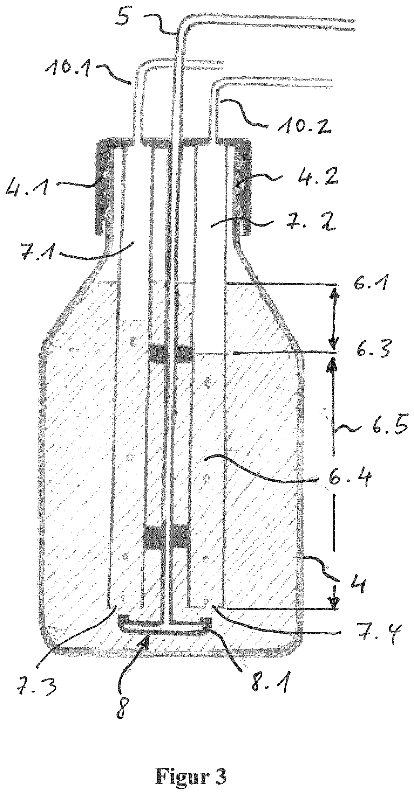

Figur 3 zeigt eine mögliche Ausführungsform eines Behälters (4). Der Behälter (4) ist als Flasche ausgebildet, deren obere Öffnung (4.2) mit einem schraubbaren Deckel (4.1) verschließbar ist. Aus der Figur ist erkennbar, dass sich die Zufuhrleitung (5) und die Steigleitungen (7.1, 7.2) in der Flüssigkeitsfüllung (6) in Richtung einer Senkrechten zu dem Flüssigkeitsspiegel (6.1) erstrecken. Um die Schüttelbewegung zu kompensieren, sind die als Rohre ausgebildeten Steigleitungen (7.1, 7.2) kreisförmig um die Zufuhrleitung (5) an dem Deckel (4.1) befestigt. Unmittelbar mit dem Aufschrauben des Deckels (4.1) auf dem Behälter (4) sind sämtliche Steigleitungen (7.1, 7.2), der sich in den Behälter (4) erstreckende Abschnitt der Zufuhrleitung (5) und der mit der Zufuhrleitung (5) fluidleitend verbundene Verteiler (8) korrekt innerhalb des Behälters (4) positioniert und zueinander ausgerichtet.

Der Verteiler (8) weist mehrere sich von der Zufuhrleitung (5) sternförmig nach außen erstreckende Verteilerrohre (8.1) auf. Sofern um die Zufuhrleitung (5) kreisförmig sechs Steigleitungen (7.1, 7.2) angeordnet sind, weist der Verteiler (8) sechs Verteilerrohre (8.1) auf. Jedes Verteilerrohr (8.1) mündet endseitig in einer im Durchmesser reduzierten Austrittsöffnung, die unterhalb der unteren Öffnung (7.3, 7.4) einer der Steigleitungen (7.1, 7.2) innerhalb der Flüssigkeitsfüllung (6) mündet. In dem dargestellten Ausführungsbeispiel befinden sich die Austrittsöffnungen knapp unterhalb der unteren Öffnungen (7.3, 7.4). Die radiale Erstreckung sämtlicher Verteilerrohre (8.1), die Geometrie der Austrittsöffnungen an den Enden der Verteilerrohre (8.1) sowie die Lage der Austrittsöffnungen zu den unteren Öffnungen (7.3, 7.4) der Steigleitungen stimmen überein, sodass übereinstimmende Teilgasströme aus dem Verteiler (8) unter übereinstimmenden Bedingungen in die Steigleitungen (7.1, 7.2) eintreten.

**Bezugszeichenliste**

| Nr. | Bezeichnung |
|---|---|
| 1. | Begasungssystem |
| 2.1 | Bioreaktor |
| 2.2 | Bioreaktor |
| 3. | Gasversorgung |
| 3.1 | Gasquellen |
| 3.2 | Eingang |
| 3.3 | Ausgang |
| 3.4 | Massendurchflussregler |
| 3.5 | Mischeinrichtung |
| 4 . | Behälter |
| 4.1 | Deckel |
| 4.2 | Öffnung |
| 5. | Zufuhrleitung |
| 6. | Flüssigkeitsfüllung |
| 6.1 | Flüssigkeitsspiegel |
| 6.3 | Flüssigkeitsoberfläche |
| 6.4 | Flüssigkeitssäule |
| 6.5 | Höhe der Flüssigkeitssäule |
| 7.1 | Steigleitungen |
| 7.2 | Steigleitungen |
| 7.3 | Untere Öffnung |
| 7.4 | Untere Öffnung |
| 7.5 | Obere Öffnung |
| 7.6 | Obere Öffnung |
| 8. | Verteiler |
| 8.1 | Verteilerrohre |
| 9.1 | Teilgasstrom |
| 9.2 | Teilgasstrom |

(fortgesetzt)

| Nr. | Bezeichnung |
|---|---|
| 10.1 | Gasleitung |
| 10.2 | Gasleitung |
| 11. | Abgasfilter |
| 12. | Überdruckventil |
| 13. | Tisch |
| 14. | Rotationsschüttler |

**Patentansprüche**

1. Verfahren zur Begasung von mehreren Bioreaktoren (2.1, 2.2) umfassend folgende Schritte

   - Bereitstellung eines konstanten Gasstroms,
   - Zuführen des konstanten Gasstroms in einen Behälter (4) mit einer Flüssigkeitsfüllung (6),
   - Aufteilen des Gasstroms in mehrere Teilgasströme (9.1, 9.2),
   - Einleiten jedes Teilgasstroms (9.1, 9.2) in die Flüssigkeitsfüllung (6), wobei jeder Teilgasstrom (9.1, 9.2) in eine separate, eine untere Öffnung (7.3, 7.4) aufweisende Steigleitung (7.1, 7.2) für jeden Teilgasstrom (9.1, 9.2) eingeleitet wird und jede Steigleitung (7.1, 7.2) in die Flüssigkeitsfüllung (6) eintaucht,

   und

   - Zuführen des in jeder Steigleitung (7.1, 7.2) aufsteigenden Teilgasstroms (9.1, 9.2) zu dem Innenraum eines der Bioreaktoren (2.1, 2.2) über eine an eine obere Öffnung (7.5, 7.6) jeder Steigleitung (7.1, 7.2) gasdicht angeschlossene Gasleitung (10.1, 10.2) .

2. Verfahren zur Begasung von mehreren Bioreaktoren nach Anspruch 1, **dadurch gekennzeichnet, dass** der Gasstrom in Teilgasströme (9.1, 9.2) mit übereinstimmendem Volumenstrom aufgeteilt wird.

3. Verfahren zur Begasung von mehreren Bioreaktoren nach Anspruch 1 oder 2, **dadurch gekennzeichnet, dass** das Einleiten jedes Teilgasstroms (9.1, 9.2) in die Flüssigkeitsfüllung (6) in übereinstimmendem vertikalen Abstand zum Flüssigkeitsspiegel (6.1) erfolgt.

4. Verfahren zur Begasung von mehreren Bioreaktoren nach einem der Ansprüche 1 bis 3, **dadurch gekennzeichnet, dass** das Einleiten jedes Teilgasstroms (9.1, 9.2) in eine der Steigleitungen (7.1, 7.2) in übereinstimmendem vertikalen Abstand zum Flüssigkeitsspiegel (6.1) erfolgt.

5. Verfahren zur Begasung von mehreren Bioreaktoren nach einem der Ansprüche 1 bis 4, **dadurch gekennzeichnet, dass** die Bioreaktoren (2.1, 2.2) und der Behälter (4) zumindest während der Begasung geschüttelt werden.

6. Begasungssystem (1) zur Begasung von mehreren Bioreaktoren (2.1, 2.2) umfassend

- eine Gasversorgung (3) zur Bereitstellung eines konstanten Gasstroms in einer Zufuhrleitung (5),
- einen Behälter (4) mit einer Flüssigkeitsfüllung (6),
- mehrere in die Flüssigkeitsfüllung (6) eintauchende Steigleitungen (7.1, 7.2), wobei jede Steigleitung eine untere Öffnung (7.3, 7.4) aufweist,
- einen in der Flüssigkeitsfüllung (6) fluidleitend mit der Zufuhrleitung (5) verbundenen Verteiler (8), der zum Aufteilen des Gasstroms in mehrere Teilgasströme (9.1, 9.2) und zum Einleiten jedes Teilgasstroms (9.1, 9.2) in die Flüssigkeitsfüllung und eine der Steigleitungen (7.1, 7.2) eingerichtet ist,
- eine an eine obere Öffnung (7.5, 7.6) jeder Steigleitung (7.1, 7.2) gasdicht angeschlossene Gasleitung (10.1, 10.2), die fluidleitend mit einem Innenraum eines der Bioreaktoren (2.1, 2.2) verbunden ist.

7. Begasungssystem (1) zur Begasung von mehreren Bioreaktoren nach Anspruch 6, **dadurch gekennzeichnet, dass** die Gasversorgung (3) ein Gasmischsystem umfasst, dessen Eingänge (3.2) mit mehreren Gasquellen (3.1) und dessen Ausgang (3.3) mit der Zufuhrleitung (5) verbunden sind.

8. Begasungssystem (1) zur Begasung von mehreren Bioreaktoren nach Anspruch 7, **dadurch gekennzeichnet, dass** das Gasmischsystem einen Massendurchflussregler (3.4) für jede Gasquelle (3.1) aufweist.

9. Begasungssystem (1) zur Begasung von mehreren Bioreaktoren nach einem der Ansprüche 6 bis 8, **dadurch gekennzeichnet, dass** jeder Bioreaktor (2.1, 2.2) sowie der Behälter (4) auf einem Schütteltisch (13) eines Rotationsschüttlers (14) angeordnet sind.

10. Begasungssystem (1) zur Begasung von mehreren Bioreaktoren nach einem der Ansprüche 6 bis 9, **dadurch gekennzeichnet, dass** sich die Zufuhrleitung (5) und die Steigleitungen (7.1, 7.2) in der Flüssigkeitsfüllung (6) senkrecht zu dem Flüssigkeitsspiegel (6.1) erstrecken.

11. Begasungssystem (1) zur Begasung von mehreren Bioreaktoren nach Anspruch 9 und 10, **dadurch gekennzeichnet, dass** die als Rohre ausgeführten Steigleitungen (7.1) kreisförmig um eine Senkrechte zu dem Flüssigkeitsspiegel (6.1) angeordnet sind.

12. Begasungssystem (1) zur Begasung von mehreren Bioreaktoren nach Anspruch 11, **dadurch gekennzeichnet, dass** die Senkrechte und eine Längsmittelachse der Zufuhrleitung (5) zusammenfallen, der Verteiler (8) mehrere sich von der Zufuhrleitung (5) sternförmig nach außen erstreckende Verteilerrohre (8.1) aufweist, wobei jedes Verteilerrohr fluidleitend mit der Zufuhrleitung (5) verbunden ist und eine Austrittsöffnung oder eine Gruppe von Austrittsöffnungen aufweist, die unterhalb der unteren Öffnung (7.3, 7.4) oder innerhalb einer der Steigleitungen (7.1, 7.2) in der Flüssigkeitsfüllung mündet.

13. Begasungssystem (1) zur Begasung von mehreren Bioreaktoren nach Anspruch 11, **dadurch gekennzeichnet, dass** die Senkrechte und eine Längsmittelachse der Zufuhrleitung (5) zusammenfallen, der Verteiler (8) einen fluidleitend mit der Zufuhrleitung (5) verbundenen hohlzylindrischen Gassammler aufweist, an dessen oberer Stirnseite mehrere Lochblenden angeordnet sind, wobei jede Lochblende unterhalb der unteren Öffnung (7.3, 7.4) einer der Steigleitungen (7.1, 7.2) in der Flüssigkeitsfüllung (6) mündet.

14. Begasungssystem (1) zur Begasung von mehreren Bioreaktoren nach Anspruch 13, **dadurch gekennzeichnet, dass** die Längsmittelachse der Zufuhrleitung (5) und des Gassammlers zusammenfallen, sämtliche Lochblenden geometrisch übereinstimmen und in übereinstimmendem radialen Abstand zu der Längsmittelachse in dem Gassammler angeordnet sind.

15. Begasungssystem (1) zur Begasung von mehreren Bioreaktoren nach einem der Ansprüche 10 bis 14 , **dadurch gekennzeichnet, dass** der Behälter (4) eine mit einem Deckel (4.1) verschließbare Öffnung aufweist und die Zufuhrleitung (5) und die Steigleitungen (7.1, 7.2) an dem Deckel (4.1) als Baugruppe befestigt sind.

16. Begasungssystem (1) zur Begasung von mehreren Bioreaktoren nach einem der Ansprüche 6 bis 15 , **dadurch gekennzeichnet, dass** jeder Bioreaktor ein Überdruckventil (12) und/oder einen Abgasfilter (11) aufweist.

17. Begasungssystem (1) zur Begasung von mehreren Bioreaktoren nach einem der Ansprüche 6 bis 16, **dadurch gekennzeichnet, dass** der Behälter (4) und die Steigleitungen (7.1, 7.2) zumindest teilweise aus transparentem Material bestehen.

## Claims

1. Method for gassing a plurality of bioreactors (2.1, 2.2) comprising the following steps

   - providing a constant gas flow,
   - supplying the constant gas flow into a container (4) with a liquid filling (6),
   - dividing the gas flow into a plurality of partial gas flows (9.1, 9.2),
   - introducing each partial gas flow (9.1, 9.2) into the liquid filling (6), wherein each partial gas flow (9.1, 9.2) is introduced into a separate riser (7.1, 7.2) having a lower opening (7.3, 7.4) for each partial gas flow (9.1, 9.2) and each riser (7.1, 7.2) dips into the liquid filling (6),

   and

   - supplying the ascending partial gas flow (9.1, 9.2) in each riser (7.1, 7.2) to the interior of one of the bioreactors (2.1, 2.2) via a gas line (10.1, 10.2) connected in a gastight manner to an upper opening (7.5, 7.6) of each riser (7.1, 7.2).

2. Method for gassing a plurality of bioreactors according to Claim 1, **characterized in that** the gas flow is divided into partial gas flows (9.1, 9.2) with the same volume flow.

3. Method for gassing a plurality of bioreactors according to Claim 1 or 2, **characterized in that** the introduction of each partial gas flow (9.1, 9.2) into the liquid filling (6) is made at the same vertical distance from the liquid level (6.1).

4. Method for gassing a plurality of bioreactors according to one of Claims 1 to 3, **characterized in that** the introduction of each partial gas flow (9.1, 9.2) into one of the risers (7.1, 7.2) is made at the same vertical distance from the liquid level (6.1).

5. Method for gassing a plurality of bioreactors according to one of Claims 1 to 4, **characterized in that** the bioreactors (2.1, 2.2) and the container (4) are shaken at least during gassing.

6. Gassing system (1) for gassing a plurality of bioreactors (2.1, 2.2) comprising

   - a gas supply (3) for providing a constant gas flow in a supply line (5),
   - a container (4) with a liquid filling (6),
   - a plurality of risers (7.1, 7.2) dipping into the liquid filling (6), wherein each riser has a lower opening (7.3, 7.4),
   - a distributor (8) connected to the supply line (5) in a fluid-conducting manner in the liquid filling (6), which is adapted for dividing the gas flow into a plurality of partial gas flows (9.1, 9.2) and for introducing each partial gas flow (9.1, 9.2) into the liquid filling and one of the risers (7.1, 7.2),
   - a gas line (10.1, 10.2) connected in a gastight manner at an upper opening (7.5, 7.6) of each riser (7.1, 7.2) which is connected in a fluid-conducting manner to an interior of one of the bioreactors (2.1, 2.2).

7. Gassing system (1) for gassing a plurality of bioreactors according to Claim 6, **characterized in that** the gas supply (3) comprises a gas mixing system whose inputs (3.2) are connected to a plurality of gas sources (3.1) and whose output (3.3) is connected to the supply line (5).

8. Gassing system (1) for gassing a plurality of bioreactors according to Claim 7, **characterized in that** the gas mixing system has a mass flow regulator (3.4) for each gas source (3.1).

9. Gassing system (1) for gassing a plurality of bioreactors according to one of Claims 6 to 8, **characterized in that** each bioreactor (2.1, 2.2) as well as the container (4) are arranged on a shaking table (13) of a rotational shaker (14).

10. Gassing system (1) for gassing a plurality of bioreactors according to one of Claims 6 to 9, **characterized in that** the supply line (5) and the risers (7.1, 7.2) in the liquid filling (6) extend perpendicularly to the liquid level (6.1).

11. Gassing system (1) for gassing a plurality of bioreactors according to one of Claims 9 and 10, **characterized in that** the risers (7.1) implemented as pipes are arranged circularly around a perpendicular to the liquid level (6.1).

12. Gassing system (1) for gassing a plurality of bioreactors according to Claim 11, **characterized in that** the perpendicular and a longitudinal central axis of the supply line (5) coincide, the distributor (8) comprises a plurality of distributor tubes (8.1) extending outwards from the supply line (5) in a star shape, wherein each distributor tube is connected in a fluid-conducting manner to the supply line (5) and has an outlet opening or a group of outlet openings which open below the lower opening (7.3, 7.4) or inside one of the risers (7.1, 7.2) in the liquid filling.

13. Gassing system (1) for gassing a plurality of bioreactors according to Claim 11, **characterized in that** the perpendicular and a longitudinal central axis of the supply line (5) coincide, the distributor (8) comprises a hollow-cylindrical gas collector connected in a fluid-conducting manner to the supply line (5), on the upper front side whereof a plurality of circular apertures are arranged, wherein each circular aperture opens in the liquid filling (6) below the lower opening (7.3, 7.4) of one of the risers (7.1, 7.2) .

14. Gassing system (1) for gassing a plurality of bioreactors according to Claim 13, **characterized in that** the longitudinal central axis of the supply line (5) and the gas collector coincide, all the circular apertures are geometrically the same and are arranged at the same radial distance from the longitudinal central axis in the gas collector.

15. Gassing system (1) for gassing a plurality of bioreactors according to one of Claims 10 to 14, **characterized in that** the container (4) has an opening which can be closed with a cover (4.1) and the supply line (5) and the risers (7.1, 7.2) are fastened to the cover (4.1) as an assembly.

16. Gassing system (1) for gassing a plurality of bioreactors according to one of Claims 6 to 15, **characterized in that** each bioreactor has a pressure relief value (12) and/or an exhaust gas filter (11).

17. Gassing system (1) for gassing a plurality of bioreactors according to one of Claims 6 to 16, **characterized in that** the container (4) and the risers (7.1, 7.2) consist at least partially of transparent material.


**Revendications**

1. Procédé d'approvisionnement en gaz de plusieurs bioréacteurs (2.1, 2.2), comprenant les étapes suivantes consistant à :

    - mettre à disposition un flux gazeux constant,
    - alimenter le flux gazeux constant dans un réservoir (4) pourvu d'une charge de remplissage liquide (6),
    - diviser le flux gazeux en plusieurs flux gazeux partiels (9.1, 9.2),
    - introduire chaque flux gazeux partiel (9.1, 9.2) dans la charge de remplissage liquide (6), chaque flux gazeux partiel (9.1, 9.2) étant introduit dans une conduite montante (7.1, 7.2) séparée, comportant une ouverture (7.3, 7.4) inférieure pour chaque flux gazeux partiel (9.1, 9.2) et chaque conduite montante (7.1, 7.2) plongeant dans la charge de remplissage liquide (6),

    et

    - alimenter le flux gazeux partiel (9.1, 9.2) s'élevant dans chaque conduite montante (7.1, 7.2) dans l'espace intérieur de l'un des bioréacteurs (2.1, 2.2) par l'intermédiaire d'une conduite de gaz (10.1, 10.2) raccordée de manière étanche au gaz sur une ouverture (7.5, 7.6) supérieure de chaque conduite montante (7.1, 7.2) .

2. Procédé d'approvisionnement en gaz de plusieurs bioréacteurs selon la revendication 1, **caractérisé en ce que** le flux gazeux est divisé en plusieurs flux gazeux partiels (9.1, 9.2) de débit volumétrique concordant.

3. Procédé d'approvisionnement en gaz de plusieurs bioréacteurs selon la revendication 1 ou 2, **caractérisé en ce que** l'introduction de chaque flux gazeux partiel (9.1, 9.2) dans la charge de remplissage liquide (6) s'effectue avec un écart vertical concordant par rapport au niveau du liquide (6.1).

4. Procédé d'approvisionnement en gaz de plusieurs bioréacteurs selon l'une quelconque des revendications 1 à 3, **caractérisé en ce que** l'introduction de chaque flux gazeux partiel (9.1, 9.2) dans l'une des conduites montantes (7.1, 7.2) s'effectue avec un écart vertical concordant par rapport au niveau du liquide (6.1).

5. Procédé d'approvisionnement en gaz de plusieurs bioréacteurs selon l'une quelconque des revendications 1 à 4, **caractérisé en ce que** les bioréacteurs (2.1, 2.2) et le réservoir (4) sont agités au moins pendant l'approvisionnement en gaz.

6. Système d'approvisionnement en gaz (1), destiné à approvisionner en gaz plusieurs bioréacteurs (2.1, 2.2) comprenant :

    - une alimentation gazeuse (3), destinée à mettre à disposition un flux gazeux dans une conduite d'alimentation (5),
    - un réservoir (4) pourvu d'une charge de remplissage liquide (6),
    - plusieurs conduites montantes (7.1, 7.2) plongeant dans la charge de remplissage liquide (6), chaque conduite montante comportant une ouverture (7.3, 7.4) inférieure,
    - un distributeur (8) qui est en liaison fluidique dans la charge de remplissage liquide (6) avec la conduite d'alimentation (5), qui est aménagé pour diviser le flux gazeux en plusieurs flux gazeux partiels (9.1, 9.2) et pour introduire chaque flux gazeux partiel (9.1, 9.2) dans la charge de remplissage liquide et dans l'une des conduites montantes (7.1, 7.2),
    - une conduite de gaz (10.1, 10.2) raccordée de manière étanche au gaz sur une ouverture (7.5, 7.6) supérieure de chaque conduite montante (7.1, 7.2), qui est en liaison fluidique avec un espace intérieur de l'un des bioréacteurs (2.1, 2.2).

7. Système d'approvisionnement en gaz (1), destiné à approvisionner en gaz plusieurs bioréacteurs selon la revendication 6, **caractérisé en ce que** l'alimentation gazeuse (3) comprend un système mélangeur de gaz, dont les entrées (3.2) sont reliées avec plusieurs sources gazeuses (3.1) et dont la sortie (3.3) est reliée avec la conduite d'alimentation (5).

8. Système d'approvisionnement en gaz (1), destiné à approvisionner en gaz plusieurs bioréacteurs selon la revendication 7, **caractérisé en ce que** le système mélangeur de gaz comporte un régulateur de débit massique (3.4) pour chaque source gazeuse (3.1).

9. Système d'approvisionnement en gaz (1), destiné à approvisionner en gaz plusieurs bioréacteurs selon l'une quelconque des revendications 6 à 8, **caractérisé en ce que** chaque bioréacteur (2.1, 2.2) ainsi que le réservoir (4) sont placés sur une table agitatrice (13) d'un agitateur rotatif (14).

10. Système d'approvisionnement en gaz (1), destiné à approvisionner en gaz plusieurs bioréacteurs selon l'une quelconque des revendications 6 à 9, **caractérisé en ce que** la conduite d'alimentation (5) et les conduites montantes (7.1, 7.2) s'étendent dans la charge de remplissage liquide (6) à la perpendiculaire du niveau du liquide (6.1).

11. Système d'approvisionnement en gaz (1), destiné à approvisionner en gaz plusieurs bioréacteurs selon la revendication 9 et 10, **caractérisé en ce que** les conduites montantes (7.1) conçues sous la forme de tuyaux sont placées en forme de cercle autour d'une verticale par rapport au niveau du liquide (6.1).

12. Système d'approvisionnement en gaz (1), destiné à approvisionner en gaz plusieurs bioréacteurs selon la revendication 11, **caractérisé en ce que** la verticale et un axe médian longitudinal de la conduite d'alimentation (5) coïncident, le distributeur (8) comporte plusieurs tuyaux distributeurs (8.1) s'étendant vers l'extérieur en forme d'étoile à partir de la conduite d'alimentation (5), chaque tuyau distributeur étant en liaison fluidique avec la conduite d'alimentation (5) et comportant une ouverture de sortie ou un groupe d'ouvertures de sortie, qui en dessous de l'ouverture (7.3, 7.4) inférieure ou à l'intérieur des conduites montantes (7.1, 7.2) débouche dans la charge de remplissage liquide.

13. Système d'approvisionnement en gaz (1), destiné à approvisionner en gaz plusieurs bioréacteurs selon la revendication 11, **caractérisé en ce que** la verticale et un axe médian longitudinal de la conduite d'alimentation (5) coïncident, le distributeur (8) comporte un collecteur de gaz cylindrique creux, qui est en liaison fluidique avec la conduite d'alimentation (5), sur la face frontale supérieure duquel sont placés plusieurs obturateurs perforés, chaque obturateur perforé débouchant en-dessous de l'ouverture (7.3, 7.4) inférieure de l'une des conduites montantes (7.1, 7.2) dans la charge de remplissage liquide (6).

14. Système d'approvisionnement en gaz (1), destiné à approvisionner en gaz plusieurs bioréacteurs selon la revendication 13, **caractérisé en ce que** l'axe médian longitudinal de la conduite d'alimentation (5) et du collecteur de gaz coïncident, tous les obturateurs perforés concordent du point de vue géométrique et sont placés dans le collecteur de gaz avec un écart radial concordant par rapport à l'axe médian longitudinal.

15. Système d'approvisionnement en gaz (1), destiné à approvisionner en gaz plusieurs bioréacteurs selon l'une quelconque des revendications 10 à 14, **caractérisé en ce que** le réservoir (4) comporte une ouverture susceptible d'être fermée par un couvercle (4.1) et la conduite d'alimentation (5) et les conduites montantes (7.1, 7.2) sont fixées en tant qu'ensemble sur le couvercle (4.1).

16. Système d'approvisionnement en gaz (1), destiné à approvisionner en gaz plusieurs bioréacteurs selon l'une quelconque des revendications 6 à 15, **caractérisé en ce que** chaque bioréacteur comporte une soupape de surpression (12) et/ou un filtre de gaz brûlés (11).

17. Système d'approvisionnement en gaz (1), destiné à approvisionner en gaz plusieurs bioréacteurs selon l'une quelconque des revendications 6 à 16, **caractérisé en ce que** le réservoir (4) et les conduites montantes (7.1, 7.2) consistent au moins partiellement en une matière transparente.

Figure 1

Figur 2

Figur 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2975110 A1
- WO 2007116266 A1